# EUROPÄISCHE PATENTSCHRIFT

(11) **EP 1 996 172 B1**
(45) Veröffentlichungstag und Bekanntmachung des Hinweises auf die Patenterteilung: **28.04.2010**
(21) Anmeldenummer: 07723077.9
(22) Anmeldetag: 07.03.2007
(51) Int. Cl.: A61K 9/70, B65D 75/30, A61F 13/02

(54) **PFLASTERVERPACKUNG**
PLASTER PACKAGING
EMBALLAGE POUR PANSEMENT

(30) Priorität: 09.03.2006 DE 102006011338
(43) Veröffentlichungstag der Anmeldung: 03.12.2008
(73) Patentinhaber: Grünenthal GmbH, 52078 Aachen (DE)
(72) Erfinder: KUGELMANN, Heinrich, 52068 Aachen (DE); BARTHOLOMÄUS, Johannes, 52080 Aachen (DE)
(74) Vertreter: Bülle, Jan
(86) Internationale Anmeldenummer: PCT/EP2007/001933
(87) Internationale Veröffentlichungsnummer: WO 2007/101660

(56) Entgegenhaltungen:
- EP-A- 1 209 097
- FR-A1- 2 848 535
- GB-A- 1 001 421

## Beschreibung

Die Erfindung betrifft eine Pflasterverpackung, welche sich gegenüber dem Stand der Technik u.a. durch eine verbesserte Handhabung und einen geringeren Materialverbrauch auszeichnet

Zur bestimmungsgemäßen Anwendung herkömmlicher Pflaster müssen typischerweise wenigstens die folgenden Schritte durchgeführt werden: Entnahme des Pflasters aus einer Verpackung (z.B. aus einem Siegelbeutel), Entfernung der die klebefähige Schicht des Pflasters bedeckenden Schutzfolie (*release liner),* Aufkleben des Pflasters auf der Haut und Entsorgung des Verpackungsmaterials (entleerte Verpackung und entfernte Schutzfolie).

Im Stand der Technik sind Pflaster bekannt, welche einzeln in Schutzhüllen verpackt sind. Die Schutzhüllen müssen unmittelbar vor der Anwendung der Pflaster z.B. durch Aufschneiden, Aufreißen oder Ziehen an einem Aufreißstreifen bzw. einer Perforation geöffnet werden. Für wirkstoffhaltige Pflaster werden an die Eigenschaften dieser Schutzhüllen besondere Anforderungen gestellt, damit eine ausreichende Lagerstabilität des Wirkstoffs gewährleistet wird und Kontaminationen verhindert werden.

Hinsichtlich der Handhabbarkeit ist diese Art der Verpackung jedoch mit Nachteilen behaftet. Insbesondere ältere oder behinderte Menschen haben Probleme, die Schutzhüllen zu öffnen. Im Falle des Aufschneidens mit Hilfe scharfer Gegenstände (Messer, Scheren, etc.) werden die Pflaster vielfach beschädigt oder sogar unbrauchbar gemacht. Zudem ist diese Art der Verpackung vergleichsweise aufwändig und kostenintensiv.

Besondere Probleme ergeben sich, wenn das Material der klebefähigen Schicht des Pflasters ein gewisses Fließverhalten (sog. *"cold flow")* zeigt. Dieses Fließverhalten ist in der Regel zwar nur sehr gering ausgeprägt, kann bei Lagerung der Pflaster über mehrere Monate jedoch sehr wohl in Erscheinung treten und Probleme bereiten.

Das Fließverhalten hat nämlich zur Folge, dass das Material der klebefähigen Schicht mit der Zeit seitlich am Rand des Pflasters austritt, dadurch In Kontakt mit der Schutzhülle kommt und daran anhaftet. Wird die Schutzhülle an einer Seite geöffnet so kann das Pflaster Infolge der Anhaftung nicht lose entnommen werden, sondern es muss zunächst aktiv die unbeabsichtigte Fixierung gelöst werden. Insbesondere ältere oder behinderte Menschen haben dann Probleme, das Pflaster aus der Verpackung zu entnehmen.

Es besteht ein Bedarf an Pflasterverpackungen, welche den Materialeinsatz reduzieren und eine Entnahme der Pflaster erleichtern.

DE-A 100 56 234 offenbart eine Primärverpackung für medizinische Pflaster, welche eine flächenförmige Packstoffbahn, die zumindest in einem Teilbereich selbstklebend ausgerüstet ist, und eine haubenförmige, weitgehend verformungsstabile Abdeckung umfasst. Die Packstoffbahn und die Abdeckung sind in der Weise miteinander verbunden, dass ein für die Verpackung eines Pflasters geeigneter, allseitig umschlossener Innenraum gebildet wird, wobei der klebende Bereich der Packstoffbahn zum Innenraum hin gewandt Ist und der Befestigung des zu verpackenden Pflasters über die Rückseite seiner Rückschicht dient So ist es möglich, auf eine die Klebefläche bedeckende Schicht zu verzichten. Stattdessen ist es jedoch erforderlich, die Packstoffbahn zumindest in einem Teilbereich selbstklebend auszurüsten, was mit zusätzlichen Herstellungsschritten verbunden ist.

GB-A 1 001 421, FR-A-2 848 535 und EP-A-1 209 097 offenbaren Verpackingen von Pflastern.

Der Erfindung liegt die Aufgabe zugrunde, eine Pflasterverpackung bereitzustellen, welche Vorteile gegenüber dem Stand der Technik aufweist. Die Entnahme der Pflaster sollte vereinfacht sein und der Materialeinsatz sollte gering sein. Insbesondere sollte das Auftreten eines "*cold flow"* des Materials der klebefähigen Schicht die Entnahme des Pflasters aus der Verpackung nicht negativ beeinflussen.

Diese Aufgabe wird durch den Gegenstand der Patentansprüche gelöst.

Es wurde überraschend gefunden, dass vorteilhafte Verpackungen hergestellt werden können, wenn das Pflaster über seine klebefähige Schicht an der Außenwand der Verpackung haftet, d.h. an der Oberfläche der Außenwand, welche dem Innenraum der Verpackung zugewandt ist. Auf diese weise kann auf den sonst üblichen, die Klebefähige Schicht bedeckenden Schutzfilm (*release liner)* verzichtet werden, da die Außenwand der Verpackung dessen Funktion übernimmt. So wird der Schutzfilm in die Verpackung integriert und dadurch der Materlaleinsatz reduziert. Mit der Entnahme des Pflasters aus der Verpackung wird gleichzeitig dessen klebefähige Schicht freigelegt.

Die Erfindung betrifft demnach eine Verpackung, in deren Innenraum mindestens ein wirkstoffhaltiges Pflaster angeordnet ist, welches über seine klebefähige Schicht an einer dem Innenraum zugewandten Oberfläche einer Außenwand der Verpackung haftet und davon ablösbar ist, wobei die Verpackung wenigstens in einem Bereich transparent ist und auf das Pflaster Informationen als Kennzeichnungen aufgedrückt sind, welche durch die transparente Ausgestaltung der Verpackung nach aussen sichtbar sind.

Die Verpackung ist wenigstens in einem Bereich transparent. Vorzugsweise ist die Fläche des transparenten Bereichs mindestens so groß wie die Fläche des Pflastern, im Innenraum der erfindungsgemäßen Verpackung, vorzugsweise größer. Bevorzugt kann die vollständig Fläche des Pflasters von außen durch den transparenten Bereich hindurch mit bloßem Auge betrachtet werden. Dazu ist wenigstens eine Außenwand der Verpackung wenigstens In einem Bereich, vorzugsweise vollständig, aus einem transparenten Material gebildet.

Es wurde überraschend befunden, dass die erfindungsgemäßen Verpackungen mehrere Vorteile auf sich vereinen. So kann einerseits der Materialeinsatz verringert werden, da auf einen separaten Schutzfilm (*release liner)* für die klebefähige Schicht des Pflasters verzichtet wird. Andererseits wirkt sich ein möglicherweise zu beobachtender "*cold flow*" nicht negativ auf die Handhabung der Verpackung, insbesondere die Entnahme des Pflasters aus.

Durch die transparente Ausgestaltung der Verpackung wird verhindert, dass beim Öffnen der Verpackung, beispielsweise mit Hilfe einer Schere, das Pflaster versehentlich beschädigt wird. So ermöglicht es der transparente Bereich, die Schere sicher am Pflaster vorbeizuführen.

Darüber hinaus ermöglicht die transparente Ausgestaltung der Verpackung, Informationen nach außen sichtbar zu machen. Diese Informationen sind als Kennzeichnungen auf das Pflaster oder auf die Oberfläche der Außenwand, welche dem Innenraum der Verpackung zugewandt ist, aufgedruckt. Durch die Verpackung werden diese Kennzeichnungen vor äußeren mechanischen Einflüssen geschützt. Zur Kennzeichnung kommen insbesondere Symbole, Zahlen und/oder Buchstaben in Frage. Als Kennzeichnungen eignen sich insbesondere Produktbezeichnungen (z.B. Markennamen), Tageszeiten (z.B. morgens, mittags, abends, nachts), Uhrzeiten, Namen von Wochentagen oder deren Abkürzungen. Namen von Monaten oder deren Abkürzungen, Wirkstoffnamen (z.B. Buprenorphin) und Wirkstoffdosierungen (z.B. 250 mg, 500 mg) bzw. Frelsetzungsraten (z.B. 35 µg/h, 70 µg/h).

Bei der erfindungsgemäßen Verpackung handelt es sich bevorzugt um eine Aufreißverpackung, welche vorzugsweise zum einmaligen Öffnen vorgesehen ist und nach der Entnahme des darin enthaltenen Pflasters entsorgt wird. Durch das ordnungsgemäßen Öffnen der Verpackung wird vorzugsweise der Blick auf die gesamte, äußere Oberfläche des Pflasters eröffnet, welche der klebefähigen Schicht abgewandt ist.

Das Pflaster ist zur Anwendung beim Patienten bestimmt, d.h. es erfüllt die dafür aus hygienischer und pharmazeutischer Sicht erforderlichen Anforderungen.

Das Pflaster ist wirkstoffhaltig. Dabei enthält es im verpackten Zustand bevorzugt die ursprüngliche, zur Verabreichung an den Patienten vorgesehene Wirkstoffmenge, d.h. das wirkstoffhaltige Pflaster wurde noch nicht beim Patienten angewendet.

Zum Zwecke der Beschreibung umfasst der Begriff "wirkstoffhaltiges Pflaster" eine selbstklebend ausgerüstete Vorrichtung, welche als Wirkstoff wenigstens eine physiologisch wirksame Substanz enthält Als Wirkstoffe kommen insbesondere Arzneistoffe (pharmazeutische Wirkstoffe), aber beispielsweise auch Nahrungsergänzungsmittel in Betracht

Der Wirkstoff kann einerseits dazu befähigt sein, die Haut zu durchdringen und In die Blutbahn aufgenommen zu werden, so dass es sich bei dem erfindungsgemäßen wirkstoffhaltigen Pflaster in diesem Fall um ein transdermales therapeutisches System (TTS) handelt. In diesem Fall erfolgt die Wirkung des Wirkstoffs systemisch. Andererseits kann der Wirkstoff jedoch auch nicht dazu befähigt sein, die Haut zu durchdringen. In diesem Fall erfolgt die Wirkung topisch bzw. lokal.

Das wirkstoffhaltige Pflaster, vorzugsweise seine klebefähige Schicht, kann als Wirkstoff eine oder mehrere, vorzugsweise transdermal verabreichbare, physiologisch wirksame Substanzen enthalten, beispielsweise Arznelstoffe (pharmazeutische Wirkstoffe), aber auch Nahrungs- oder Nahrungsergänzungsmittel. Der Wirkstoff kann systemisch oder topisch wirksam sein. Vorzugsweise enthält das wirkstoffhaltige Pflaster wenigstens einen transdermal verabreichbaren Wirkstoff, welcher systemisch wirksam ist. Der Wirkstoff kann dabei in verschiedenen physikalischen Zuständen vorliegen, wie molekular verteilt, als Kristalle, in Form von Clustern oder eingekapselt in Liposomen.

Beispiele für vorzugsweise transdermal verabreichbare Nahrungs- oder Nahrungsergänzungsmittel sind Proteine, Saccharide, Lipide, Vitamine, Provitamine, Spurenelemente, gesättigte oder ungesättigte Fettsäuren und Antioxidanzien.

Bevorzugt enthält das wirkstoffhaltige Pflaster einen oder mehrere Wirkstoffe, vorzugsweise allerdings nur einen einzigen transdermal verabreichbaren Wirkstoff. Der Wirkstoff ist vorzugsweise zumindest zum Teil in eine Matrix eingebettet.

Vorzugsweise ist der Wirkstoff ausgewählt aus der Gruppe bestehend aus Antiallergika; Antiarthritika; Antiasthmatika; Antibiotika und anderen antimikrobiellen Mitteln, wie Tetracyclinen, Oxytetracyclinen, Chlortetracyclinen, Sulfonamiden; Antidepressiva; Antidiabetika, wie Insulin; Antiepileptika; Antihistaminika; Antihypertonika; Anti-Krampfmitteln, wie Atropin, Butylscopolamin-Bromid; Antimigränemitteln; Antipyretika; Antirheumatika; antiviralen Mitteln; Anxiolytika; Cardiaca; cardiovasculären Verbindungen, wie Nitroglycerin, cardialen Glycosiden; Coronardilatatoren; Corticosteroiden; entzündungshemmenden Mitteln; Enzymen; Fungiziden; Immunmodulatoren; Impfstoffen; Kontrazeptiva; Lokalanästhetika; Mitteln zur Behandlung von Alkohol- und/oder Drogen- und/oder Medikamentenabhängigkeit; Mitteln zur Behandlung von Erkrankungen des zentralen Nervensystems; Mitteln zur Behandlung von neurodegenerativen Erkrankungen, insbesondere zur Behandlung von Morbus Parkinson und/oder Morbus Alzheimer; Narkotika und Analgetika, wie Benzocain, Lidocain, Prilocain; nicht steriodalen Antirheumatika oder entzündungshemmenden Verbindungen, wie Indometacin, Diclofenac; Nicotin; Opioiden (einschließlich Opiaten); Psychopharmaka, wie 3-(2-Aminopropyl)indolacetat und 3-(2-Aminobutyl)indolacetat; Sedative wie Pentabarbiturat-Natrium, Phenobarbiturat, Secobarbiturat-Natrium, Codein, Carbromat; (Sexual-)Hormonen, wie Adrenocorticosteroiden, wie 6-α-Methylprednisolon, androgenen Steroiden, wie Methyltestosteroide, Fluoxymesteron, östrogenen Steroiden, wie Östrogen, 17-β-Estradiol, Ethinylestradiol, Progesteron, Norethindron, Thyroxin; Stimulantien; Tranquilizern, wie Reserpin, Chlorpromazin-Hydrochlorid; und Vitaminen.

In einer bevorzugten Ausführungsform enthält das wirkstoffhaltige Pflaster einen transdermal verabreichbaren Wirkstoff, welcher bevorzugt ausgewählt ist aus der Gruppe bestehend aus Hormonen, Analgetika und Mitteln zur Behandlung neurodegenerativer Erkrankungen.

In einer bevorzugten Ausführungsform ist der Wirkstoff ausgewählt aus der Gruppe bestehend aus Narkotika, Opioiden (einschließlich Opiaten), Tranquilizern, vorzugsweise Benzodiazepinen, Stimulantien und anderen Betäubungsmitteln.

In einer bevorzugten Ausführungsform handelt es sich bei dem Wirkstoff um eine Analgetikum. Bevorzugt werden erfindungsgemäß als Analgetikum diejenigen pharmazeutischen Wirkstoffe verstanden, die von der WHO dem ATC-Index N02 zugeordnet werden (vorzugsweise in der amtlichen deutschen Fassung vom 1. Januar 2005). Bevorzugte Analgetika sind "Opioide" (ATC-Code N02A), "andere Analgetika und Antipyretika" (ATC-Code N02B) und "Migränemittel" (ATC-Code N02C). Innerhalb der bevorzugteren Opioide sind besonders bevorzugt die "natürlichen Opium-Alkaloide" (ATC-Code N02AA), "Phenylpiperidin-Derivate" (ATC-Code N02AB), "Diphenylpropylamin-Derivate" (ATC-Code N02AC), "Benzomorphan-Derivate" (ATC-Code N02AD), "Oripavin-Derivate" (ATC-Code N02AE), "Morphinan-Derivate" (ATC-Code N02AF), "Opioide in Kombination mit Spasmolytika" (ATC-Code N02AG) und "andere Opioide" (ATC-Code N02AX).

In einer besonders bevorzugten Ausführungsform ist der Wirkstoff ein Opioid. Als Opioide eignen sich besonders bevorzugt µ-, κ- oder δ-Opioidrezeptor-Agonisten, ganz besonders bevorzugt µ-Opioidrezeptor-Agonisten.

In einer bevorzugten Ausführungsform ist der Wirkstoff ein transdermal verabreichbares Opioid, Tranquilizer oder ein anderes Betäubungsmittel, das ausgewählt ist aus der Gruppe bestehend aus Alfentanil, Allobarbital, Allylprodin, Alphaprodin, Alprazolam, Amfepramon, Amfetamin, Amfetaminil, Amobarbital, Anileridin, Apocodein, Barbital, Benzylmorphin, Bezitramid, Bromazepam, Brotizolam, Buprenorphin, Butobarbital, Butorphanol, Camazepam, Cathin (D-Norpseudoephedrin), Chlordiazepoxid, Clobazam, Clonazepam, Clonitazen, Clorazepat, Clotiazepam, Cloxazolam, Cocain, Codein, Cyclobarbital, Cyclorphan, Cyprenorphin, Delorazepam, Desomorphin, Dextromoramid, Dextropropoxyphen, Dextromethorphan, Dezocin, Diampromid, Diamorphon, Diazepam, Dihydrocodein, Dihydromorphin, Dimenoxadol, Dimepheptanol, Dimethylthiambuten, Dioxaphetylbutyrat, Dipipanon, Dronabinol, Eptazocin, Estazolam, Ethoheptazin, Ethylmethylthiambuten, Ethylloflazepat, Ethylmorphin, Etonitazen, Etorphin, Fencamfamin, Fenetyllin, Fenproporex, Fentanyl, Fludiazepam, Flunitrazepam, Flurazepam, Halazepam, Haloxazolam, Heroin, Hydrocodon, Hydromorphon, Hydroxypethidin, Isomethadon, Hydroxymethylmorphinan, Ketazolam, Ketobemidon, Levacetylmethadol (LAAM)), Levomethadon, Levorphanol, Levophenacylmorphan, Levoxemacin, Lofentanil, Loprazolam, Lorazepam, Lormetazepam, Mazindol, Medazepam, Mefenorex, Meperidin, Meprobamat, Meptazinol, Metazocin, Methylmorphin, Metamfetamin, Methadon, Methaqualon, Methylphenidat, Methylphenobarbital, Methyprylon, Metopon, Midazolam, Modafinil, Morphin, Myrophin, Nabilon, Nalbuphen, Nalorphin, Narcein, Nicomorphin, Nimetazepam, Nitrazepam, Nordazepam, Norlevorphanol, Normethadon, Normorphin, Norpipanon, Opium, Oxazepam, Oxazolam, Oxycodon, Oxymorphon, Papaver somniferum, Papaveretum, Pernolin, Pentazocin, Pentobarbital, Pethidin, Phenadoxon, Phenomorphan, Phenazocin, Phenoperidin, Piminodin, Pholcodein, Phenmetrazin, Phenobarbital, Phentermin, Pinazepam, Pipradrol, Piritramid, Prazepam, Premethadion, Profadol, Proheptazin, Promedol, Properidin, Propoxyphen, Remifentanil, Secbutabarbital, Secobarbital, Sufentanil, Temazepam, Tetrazepam, Tilidin (cis und trans), Tramadol, Triazolam, Vinylbital, (1R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol, (1R, 2R)-3-(2-Dimethylaminomethylcyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1 RS, 3RS, 6RS)-6-Dimethylaminomethyl-1-(3-methoxy-phenyl)-cyclohexan-1,3-diol, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxycyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxybiphenyl-4-carbonsäure 3-(2-dimethylaminomethyl-1-hydroxy-cyclohexyl)-phenyl ester sowie deren entsprechende Stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt deren Hydrochloride.

Die Verbindungen (1 R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)-cyclohexanon oder deren stereoisomere Verbindungen oder deren physiologisch verträgliche Verbindungen, insbesondere deren Hydrochloride, deren Derivate, wie Ester, Ether oder Amide sowie Verfahren zu ihrer Herstellung sind beispielsweise aus EP-A-693 475 bzw. EP-A-780 369 bekannt. Die entsprechenden Beschreibungen werden hiermit als Referenz eingeführt und gelten als Teil der Offenbarung.

Die Wirkstoffe, ggf. in Form ihrer Ether-, Ester- oder Säurederivate, können jeweils als reines Stereoisomer, insbesondere Enantiomer oder Diastereomer, Racemat oder in Form einer Mischung von Stereoisomeren, insbesondere der Enantiomeren und/oder Diastereomeren, in einem beliebigen Mischungsverhältnis, oder jeweils in Form entsprechender physiologisch verträglicher Salze, oder jeweils in Form entsprechender Solvate vorliegen.

Als geeignete physiologisch verträgliche Salze seien beispielhaft genannt die durch Umsetzung der freien Basen des jeweiligen Wirkstoffs mit einer anorganischen oder organischen Säure, vorzugsweise mit Salzsäure, Bromwasserstoffsäure, Schwefelsäure, Phosphorsäure, Methansulfonsäure, Benzolsulfonsäure, p-Toluolsulfonsäure, Kohlensäure, Ameisensäure, Essigsäure, Propionsäure, Oxalsäure, Bernsteinsäure, Weinsäure, Mandelsäure, Fumarsäure, Maleinsäure, Milchsäure, Äpfelsäure, Sebacinsäure, Citronensäure, Ascorbinsäure, Nicotinsäure, Glutaminsäure oder Asparaginsäure erhaltenen Salze.

Als weitere physiologisch verträgliche Salze seien beispielhaft die durch Umsetzung der freien Säuren des jeweiligen Wirkstoffs mit einer geeigneten Base erhaltenen Salze genannt. Beispielhaft seien die Alkalimetallsalze, Erdalkalimetallsalze oder Ammoniumsalze [NHₓR₄₋ₓ]⁺ genannt, worin x = 0, 1, 2, 3 oder 4 ist und R für einen linearen oder verzweigten C₁₋₄-Alkyl-Rest steht.

Insbesondere bevorzugt ist das Opioid Buprenorphin (ATC-Code N02AE01) als freie Base oder in Form eines seiner pharmazeutisch verträglichen Salze, vorzugsweise Buprenorphin Hydrochlorid, Buprenorphin Saccharinat, Buprenorphin Formiat, Buprenorphin Mesylat, Buprenorphin Hydrogencitrat, Buprenorphin Nicotinat oder Buprenorphin Sebacinat.

Die erfindungsgemäße Verpackung umschließt einen Innenraum, in dem das Pflaster angeordnet ist. Dieser Innenraum wird vorzugsweise vollständig von der Außenwand der Verpackung umschlossen. Vorzugsweise ist der Innenraum gas- und wasserdicht verschlossen. Dabei reicht die Gas- bzw. Wasserdichtigkeit der Verpackung vorzugsweise aus, um den Wirkstoff, welcher ggf. in dem Pflaster enthalten ist, vor äußeren Einflüssen zu schützen, so dass eine hinreichende Lagerstabilität des Pflasters und des darin enthaltenen Wirkstoffs gewährleistet wird. Zum Zwecke der Lagerung kann die erfindungsgemäße Verpackung oder können auch mehrere der erfindungsgemäßen Verpackungen gemeinsam in eine einzige Umverpackung eingebracht werden, welche ihrerseits einen zusätzlichen Schutz vor äußeren Einflüssen bietet.

Die Außenwand der erfindungsgemäßen Verpackung kann ein- oder mehrteilig ausgebildet sein. Bevorzugt ist sie einteilig ausgebildet. Bevorzugt kann sie nach Entnahme des Pflasters aus der Verpackung als ein einzelnes, zusammenhängendes Element entsorgt werden.

In einer bevorzugten Ausführungsform wird die Außenwand der erfindungsgemäßen Verpackung aus einer ein- oder mehrschichtigen Folie gebildet. Geeignete Materia-Ilen für die Folie sind dem Fachmann bekannt und hängen u.a. von den Lagerbedingungen ab, denen die Verpackung später aufgesetzt werden soll. Bevorzugt handelt es sich bei der Folie um eine Mehrschichtfolie, wobei die einzeinen Schichten auf Materialien basieren können unabhängig voneinander ausgewählt aus der Gruppen bestehend aus Textilien, Papier, Pappe, Kunststoffen und Metall. In einer bevorzugten Ausführungsform handelt es sich um metallisiertes Papier.

Die Außenwand kann ein Polymer enthalten ausgewählt aus der Gruppe bestehend aus Polyethylen, Polyester, Polyethylenterephthalat, Polypropylen, Polysiloxan, Polyvinylchlorid und Polyurethan. Gegebenenfalls kann sie behandelte Paplerfasern oder Zellophan enthalten und/oder gegebenenfalls eine Silikon-, Fluorsililkon- oder Fluorcarbonbeschichtung aufweisen. Bevorzugt weist die Außenwand eine Dicke von 10 bis 1.000 µm, bevorzugter von 15 bis 500 µm, noch bevorzugter 20 bis 250 µm und insbesondere 25 bis 100 µm auf.

Vor der Anwendung des wirkstoffhaltigen Pflasters wird dieses von der Außenwand abgelöst. Dadurch wird die Oberfläche der klebefähigen Schicht freigelegt. Bevorzugt grenzt die Oberfläche der Außenwand unmittelbar an die klebefähige Schicht des wirkstoffhaltigen Pflasters an.

Die Verpackung ist wenigstens in einem Bereich transparent. Der Begriff "transparent" im Sinne der Erfindung bedeutet, dass ein verpacktes Pflaster durch die Verpackung hindurch mit bloßem Auge betrachtet werden kann. Die Transparenz wird bevorzugt mit Hilfe von Densitometern quantifiziert. Derartige Methoden sind dem Fachmann geläufig. Bevorzugt kann als Maß für die Transparenz die Trübung als optischer Wert gemessen werden. Die Messung der Trübung erfolgt bevorzugt nach der ASTM-Prüfnonn D 1003-61 m, Procedure A. Die erfindungsgemäße Verpackung weist bevorzugt wenigstens in einem Teilbereich eine Trübung von weniger als 30%, bevorzugter weniger als 25%, noch bevorzugter weniger als 20%, am bevorzugtesten weniger als 15% und Insbesondere weniger als 12% auf.

Geeignete Materialien zur Herstellung transparenter Verpackungen sind dem Fachmann bekannt. In diesem Zusammenhang kann beispielsweise vollumfänglich verwiesen werden auf L. Massey, Permeability Properties of Plastics and Elastomers: A Guide to Packaging and Barrier Materials; Plastics Design Library; 2nd ed, 2003; H. Lockhart et al., Packaging Pharmaceutical and Healthcare Products, Springer; 1 ed, 2006; und D.A. Dean, Pharmaceutical Packaging Technology, Taylor & Francis; 1 ed, 2000.

Um den Schutz vor UV-Strahlung zu verbessern, kann der transparente Bereich eingefärbt sein. Um eine ausreichende Luftdichtigkeit zu erzielen, kann der transparente Bereich mehrschichtig ausgebildet sein und eine Barriereschicht umfassen, welche vorzugsweise auf (Co-)-Polyamid, Ethylen-Vinylalkohol-Copolymerisat (EVOH) oder Polyvinylidenchlorid basiert.

Bevorzugt kann die gesamte Fläche des Pflasters, welche seiner klebefähigen Schicht abgewandt ist, durch den transparenten Bereich der Verpackung hindurch mit bloßem Auge betrachtet werden. Besonders bevorzugt ist der transparente Bereich auf seiner dem Innenraum der Verpackung zugewandten Oberfläche mit einer klebefähigen Schicht ausgerüstet, welche an derjenigen Fläche des Pflasters haftet, die dessen klebefähiger Schicht abgewandt ist.

Handelt es sich bei der Außenwand um eine Mehrschichtfolie, so können die einzelnen Schichten durch herkömmliche Methoden miteinander verbunden sein. Beispielsweise können die einzelnen Schichten durch Co-Extrusion, Kaschierung, Extrusionskaschierung und/oder Extrusionslaminierung zusammengefügt sein. Eine Metallisierung kann durch Bedampfung im Hochvakuum erfolgen. Derartige Herstellungsverfahren sind dem Fachmann bekannt.

Die dem Innenraum der Verpackung zugewandte Oberfläche der Außenwand der Verpackung kann voll- oder teilflächig silikonisiert oder fluoridiert sein, um das Ablösen des daran haftenden Pflasters zu vereinfachen.

In einer bevorzugten Ausführungsform ist das Pflaster mit einer vorzugsweise transparenten Abdeckfolie bedeckt. Das Pflaster befindet sich dann zwischen der Oberfläche der Außenwand der Verpackung und der Abdeckfolie. Die Fläche der vorzugsweise transparenten Abdeckfolie ist vorzugsweise größer als die Fläche des Pflasters und weist vorzugsweise wenigstens in ihren Randbereichen einen druckempfindlichen Klebstoff auf. Über diesen Randbereich haftet die Abdeckfolie auf der Oberfläche der Außenwand der Verpackung. Vorzugsweise weist der gesamte Randbereich der Abdeckfolie einen druckempfindlichen Klebstoff auf, so dass nach dem Anhaften der Abdeckfolie an der Oberfläche der Außenwand der Verpackung ein geschlossener Hohlraum aus Abdeckfolie und Außenwand gebildet wird, in dem das Pflaster angeordnet ist. Dieser Hohlraum befindet sich dann seinerseits im Innenraum der Verpackung. Die Abdeckfolie kann mit einer Lasche oder einer anderen Vorrichtung ausgerüstet sein, die geeignet ist, das Abziehen der Abdeckfolie von der Oberfläche der Außenwand zu vereinfachen.

Die vorzugsweise transparente Abdeckfolie kann als zusätzlicher Schutz für das Pflaster dienen, beispielsweise wenn das Pflaster bzw. ein ggf. darin enthaltener Wirkstoff feuchtigkeitsempfindlich ist, oder in besonderer Weise vor äußeren mechanischen Einwirkungen geschützt werden muss. Ist die Abdeckfolie nicht nur in ihren Randbereichen, sondern z.B. vollflächig selbstklebend ausgerüstet, so kann sie auch auf der Oberflächenseite des Pflasters haften, welche der klebefähigen Schicht abgewandt ist. In diesem Fall kann die Abdeckfolie ggf. als Applikationshilfe verwendet werden.

In einer bevorzugten Ausführungsform der erfindungsgemäßen Verpackung ist das Pflaster auf seiner Oberflächenseite, welche der klebefähigen Schicht abgewandt ist, mit einer selbstklebenden und ablösbaren, vorzugsweise transparenten Applikationshilfe versehen. Die Applikationshilfe kann mehrteilig sein, vorzugsweise ist sie zweiteilig. Vorzugsweise ist die Applikationshilfe transparent. Vorzugsweise ist die Haftkraft der Applikationshilfe auf der Oberflächenseite des Pflasters, welche der klebefähigen Schicht abgewandt ist, größer als die Haftkraft der klebefähigen Schicht des Pflasters auf der Oberfläche der Außenwand der Verpackung. Auf diese Weise wird gewährleistet, dass bei Ausübung einer Zugkraft auf die Applikationshilfe diese mitsamt dem Pflaster von der Oberfläche der Außenwand der Verpackung abgelöst wird, also nicht das Pflaster auf der Oberfläche der Außenwand der Verpackung zurückbleibt.

Die Funktionsweise der vorzugsweise transparenten Applikationshilfe kann darauf beruhen, dass sie aus einem Material gebildet ist, welches eine höhere Rigidität als das Pflaster aufweist. Auf diese Weise wird die Handhabung des Pflasters beim Ablösen von der Oberfläche der Außenwand der Verpackung und Aufbringen auf der Haut vereinfacht. Auch die Gefahr, dass der Patient bzw. eine Pflegeperson bei der Applikation des Pflasters versehentlich mit den Fingern die ggf. vorhandene, wirkstoffhaltige Schicht berührt, kann durch die Applikationshilfe vermindert werden.

Die Größe von wirkstoffhaltigen Pflastern wird mit zunehmendem wissenschaftlichen Fortschritt immer geringer, so dass es auch immer schwieriger wird, wirkstoffhaltige Pflaster mit einem wirkstofffreien Rand zu umgeben. Die Gefahr von Kontaminationen, beispielsweise bei Pflegepersonal, welches versehentlich die wirkstoffhaltige Fläche des Pflasters berührt, kann durch die Applikationshilfe wirksam vermindert werden.

Bestimmungsgemäß wird die Applikationshilfe erst dann vom Pflaster abgelöst, wenn dieses auf der Haut des Patienten haftet. Dazu ist bevorzugt die Haftkraft der Applikationshilfe auf der Oberflächenseite des Pflasters, welche der klebefähigen Schicht abgewandt ist, kleiner als die Haftkraft der klebefähigen Schicht des Pflasters auf der menschlichen Haut.

Die Applikationshilfe ist vorzugsweise eine ggf. mehrschichtige, vorzugsweise transparente Polymerfolie, ein Papierlaminat oder eine Metallfolie.

In einer bevorzugten Ausführungsform dient eine Außenwand der erfindungsgemäßen Verpackung als Applikationshilfe, d.h. die Applikationshilfe ist integraler Bestandteil der Verpackung. Dazu umfasst die Verpackung eine erste Außenwand und eine zweite Außenwand, welche einen Innenraum bilden, in dem mindestens ein Pflaster angeordnet ist. Das Pflaster haftet über seine klebefähige Schicht an einer dem Innenraum zugewandten Oberfläche der ersten Außenwand der Verpackung und ist davon ablösbar. Bei dieser Ausführungsform ist die dem Innenraum zugewandte Oberfläche der zweiten Außenwand ebenfalls selbstklebend ausgerüstet und haftet an derjenigen Oberfläche des Pflasters, welche dessen klebefähiger Schicht abgewandt ist. Die Oberfläche des Pflasters, welche dessen klebefähiger Schicht abgewandt ist, ist üblicherweise die äußere Oberfläche einer Trägerschicht *(backing layer*)*,* welche bei wirkstoffhaltigen Pflastern vorzugsweise wirkstoffundurchlässig und ggf. okklusiv ist. Besonders bevorzugt ist die zweite Außenwand der Verpackung wenigstens in einem Teilbereich transparent, vorzugsweise insgesamt transparent. Die Klebkraft zwischen der zweiten Außenwand und dem Pflaster ist vorzugsweise größer als die Klebkraft zwischen der klebefähigen Schicht des Pflasters und der ersten Außenwand, so dass das Pflaster beim Auseinanderziehen der ersten Außenwand und der zweiten Außenwand an der zweiten Außenwand haften bleibt und gleichzeitig dessen klebefähige Schicht freigelegt wird.

Es wurde überraschend gefunden, dass mit Hilfe einer transparenten Applikationshilfe die Anwendung des Pflasters vereinfacht wird. So kann die Stelle der Haut, auf welcher das Pflaster angebracht werden soll durch die transparente Applikationshilfe hindurch anvisiert werden, was die Zuverlässigkeit der Positionierung deutlich erhöht. Ist die Applikationshilfe gleichzeitig eine Außenwand der Verpackung, kann Verpackungsmaterial eingespart werden.

Bei der erfindungsgemäßen Verpackung können auch mehrere Pflaster in dem Innenraum angeordnet sein. Diese mehreren Pflaster können wirkstoffhaltig sein und den Wirkstoff jeweils in unterschiedlicher Dosierung und/oder Abgaberate enthalten. In einer bevorzugten Ausführungsform weisen die mehreren Pflaster gleiche oder verschiedene Markierungen auf.

In einer bevorzugten Ausführungsform ist die erfindungsgemäße Verpackung wiederverschließbar. Realisierungsmöglichkeiten wiederverschließbarer Verpackungen sind dem Fachmann bekannt. In diesem Zusammenhang kann beispielsweise verwiesen werden auf EP-A 1 590 260, EP-A 1 500 601, EP-A 1 366 999, EP-A 1 338 525, EP-A 1 448 387 und EP-A 1 513 730.

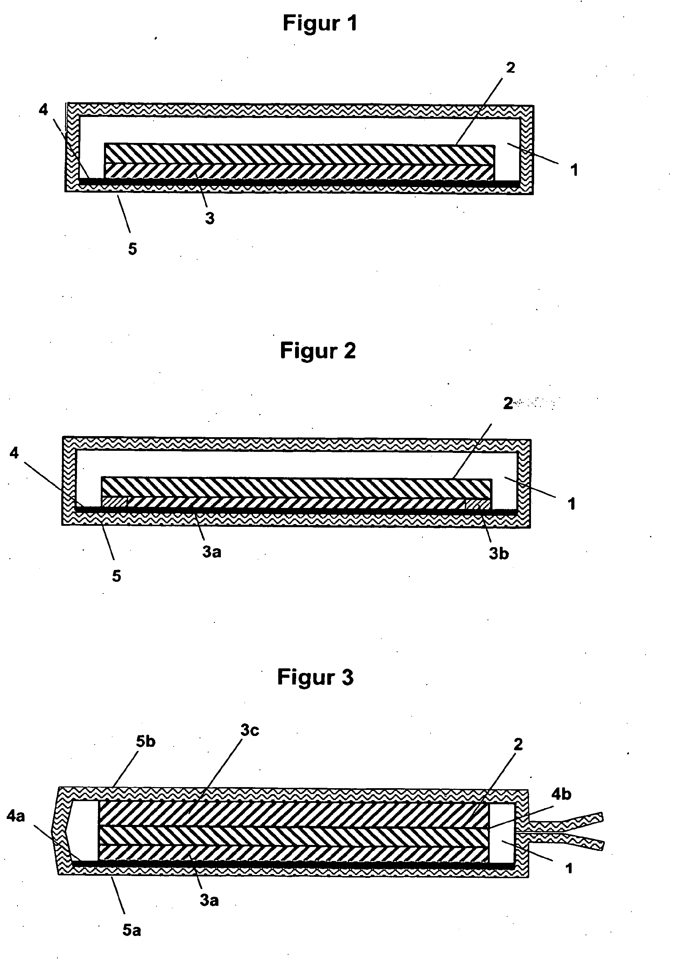

Weitere bevorzugte Ausführungsformen der erfindungsgemäßen Verpackung werden nachfolgend im Zusammenhang mit Figuren 1 bis 6 erläutert.
Figur 1 zeigt schematisch eine bevorzugte Ausführungsform der erfindungsgemäßen Verpackung in der Seitenansicht. Im Innenraum (1) der Verpackung ist ein Pflaster (2) angeordnet und haftet über seine klebefähige Schicht (3) an einer dem Innenraum (1) zugewandten Oberfläche (4) einer Außenwand (5) der Verpackung.
Figur 2 zeigt schematisch eine bevorzugte Weiterbildung der in Figur 1 abgebildeten Ausführungsform in der Seitenansicht. Bei dem Pflaster (2) handelt es sich um ein wirkstoffhaltiges Pflaster. Die klebefähige Schicht (3) ist in einen wirkstofffreisetzenden Bereich (3a) und einen wirkstofffreien Bereich (3b) unterteilt.

Dabei bedeutet "wirkstofffreisetzend" bevorzugt, dass der Wirkstoff in der klebefähigen Schicht (3) *(drug in adhesive)* und/oder in einer jenseits der klebefähigen Schicht (3) liegenden Schicht (z.B. *drug in matrix)* enthalten ist und bei Anwendung des wirkstoffhaltigen Pflasters auf der Haut eines Patienten durch den darüber liegenden Bereich der klebefähigen Schicht (3) hindurch diffundieren kann. "Wirkstofffrei" bedeutet bevorzugt, dass in diesem Bereich weder die klebefähige Schicht, noch eine jenseits der klebefähigen Schicht liegende Schicht Wirkstoff enthält.

Bevorzugt umgibt der wirkstofffreie Bereich (3b) den wirkstoffhaltigen Bereich (3a) am äußeren Rand der klebefähigen Schicht (3) wie ein Rahmen. Dies hat mitunter den Vorteil, dass der Wirkstoff, wenn der Patient bei der Applikation des Pflasters versehentlich mit den Fingerkuppen den Randbereich der klebefähigen Schicht berührt, nicht sogleich auf die Haut der Finger übertragen wird.

Figur 3 zeigt eine bevorzugte Ausführungsform der erfindungsgemäßen Verpackung, bei der eine Außenwand als Applikationshilfe dient. Dazu umfasst die Verpackung eine erste Außenwand (5a) und eine zweite Außenwand (5b), welche einen Innenraum (1) bilden, in dem mindestens ein Pflaster (2) angeordnet ist. Das Pflaster haftet über seine klebefähige Schicht (3a) an einer dem Innenraum (1) zugewandten Oberfläche (4a) der ersten Außenwand (5a) der Verpackung und ist davon ablösbar. Bei dieser Ausführungsform ist die dem Innenraum (1) zugewandte Oberfläche (4b) der zweiten Außenwand (5b) mit einer selbstklebenden Schicht (3c) ausgerüstet und haftet ablösbar an derjenigen Oberfläche des Pflasters (2), welche seiner klebefähigen Schicht (3a) abgewandt ist (*backing layer*)*.* Besonders bevorzugt ist die zweite Außenwand (5b) der Verpackung wenigstens in einem Teilbereich transparent. Die Klebkraft zwischen der zweiten Außenwand (5b) bzw. ihrer selbstklebenden Schicht (3c) und dem Pflaster ist vorzugsweise größer als die Klebkraft zwischen der klebefähigen Schicht (3a) des Pflasters und der ersten Außenwand (5a), so dass das Pflaster beim Auseinanderziehen der ersten Außenwand (5a) und der zweiten Außenwand (5b) an der zweiten Außenwand (5b) haften bleibt und gleichzeitig seine klebefähige Schicht (3a) freigelegt wird. Bevorzugt sind die Außenwände (5a) und (5b) über eine Perforation miteinander verbunden.

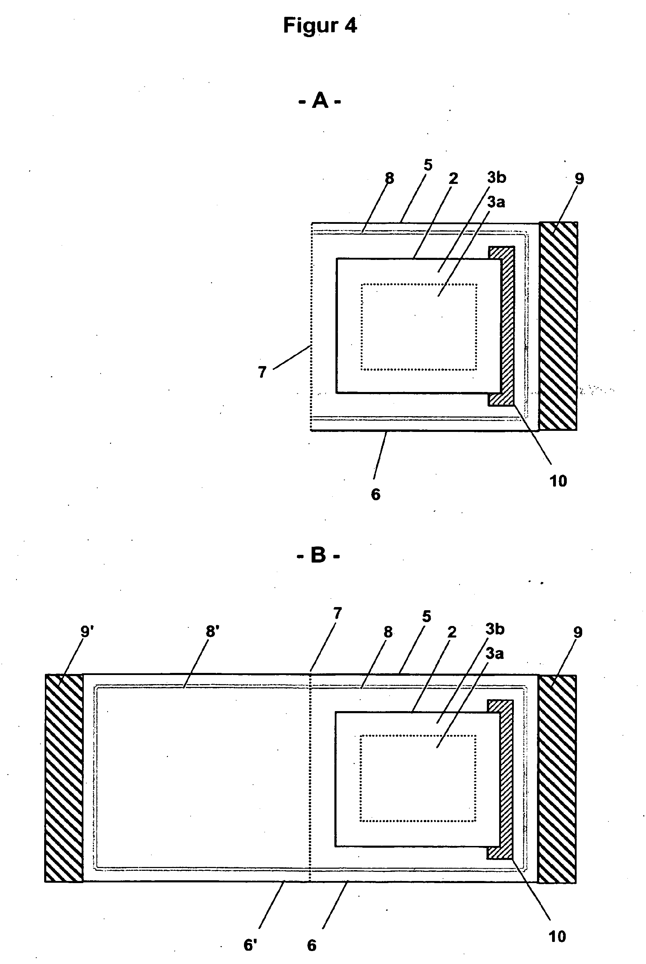

Figur 4 zeigt schematisch eine bevorzugte Ausführungsform der erfindungsgemäßen Verpackung in der Draufsicht. Figur 4A zeigt die Verpackung im geschlossenen Zustand, Figur 4B zeigt die Verpackung im geöffneten Zustand. Die Außenwand (5) umfasst zwei im wesentlichen spiegelsymmetrische Teilbereiche (6) und (6'). Die Außenwand (5) ist einteilig ausgebildet und die Teilbereiche (6) und (6') grenzen über eine Falz (7) aneinander an. Im geschlossenen Zustand liegen die Teilbereiche (6) und (6') im wesentlichen deckungsgleich übereinander und bilden dadurch den Innenraum (1).

Bevorzugt ist dazu der Randbereich der dem Innenraum (1) zugewandten Oberfläche des Teilbereichs (6) mit dem Randbereich der dem Innenraum (1) zugewandten Oberfläche des Teilbereichs (6') über eine Siegelnaht (8) verbunden. Geeignete Siegelklebstoffe sind dem Fachmann bekannt. In diesem Zusammenhang kann beispielsweise verwiesen werden auf W. Brockmann et al., Klebtechnik, Klebstoffe, Anwendungen und Verfahren, Wiley-VCH 2005.

Bevorzugt ist Teilbereich (6) und/oder Teilbereich (6') wenigstens in einem Teilbereich, vorzugsweise vollständig, aus einem transparenten Material gebildet.

Bevorzugt weist die Außenwand (5) wenigstens eine Lasche (9) zum Öffnen der Verpackung auf. In der abgebildeten Ausführungsform weist der Teilbereich (6) eine Lasche (9) und/oder der Teilbereich (6') eine Lasche (9') auf. Da die Lasche im geschlossenen Zustand der Verpackung jenseits der Siegelnaht (8) angeordnet ist, kann sie zwischen zwei Fingern festgehalten werden, wodurch das Öffnen der Verpackung erleichtert wird. So kann beispielsweise Lasche (9) kann mit der einen Hand und Lasche (9') mit der anderen Hand gefasst werden und die Siegelnaht (8) durch Ausüben einer Zugkraft gespalten werden. Dadurch wird der Teilbereich (6) vom Teilbereich (6') derart abgelöst, dass beide Teilbereiche nur noch über die Falz (7) miteinander verbunden sind. Auf diese Weise kann die erfindungsgemäße Verpackung wie ein Buch aufgeklappt werden, wobei die Falz (7) wie der Buchrücken wirkt.

In einer bevorzugten Ausführungsform ist seitlich an einem Rand des Pflasters (2) ein Streifen (10) angebracht, welcher das Ablösen der Pflasters (2) von der Oberfläche (4) vereinfacht. Der Streifen (10) ist zwischen Oberfläche (4) und der klebefähigen Schicht (3) angeordnet und verhindert dort lokal das Anhaften der klebefähigen Schicht (3) an der Oberfläche (4). Der Streifen (10) kann auf seiner Oberfläche silikonisiert oder fluoridiert sein. Der Streifen (10) kann mit den Fingern gefasst und das Pflaster (2) von der Oberfläche (4) abgezogen werden. Anschließen, ggf. nach Applikation des Pflasters auf der Haut, kann der Streifen (10) vom Rand des Pflasters (2) abgelöst und verworfen werden.

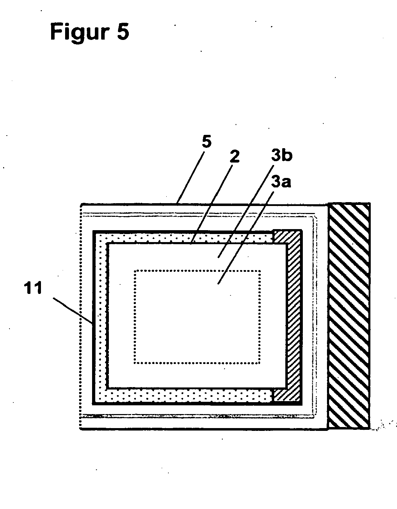

Figur 5 zeigt schematisch eine weitere bevorzugte Ausführungsform der erfindungsgemäßen Verpackung. Dabei ist die Oberfläche (4) nicht vollflächig, sondern lediglich in einem Teilbereich (11) modifiziert, beispielsweise silikonisiert oder fluoridiert. Im verpackten Zustand haftet das Pflaster auf diesem Teilbereich (11) der Oberfläche (4).

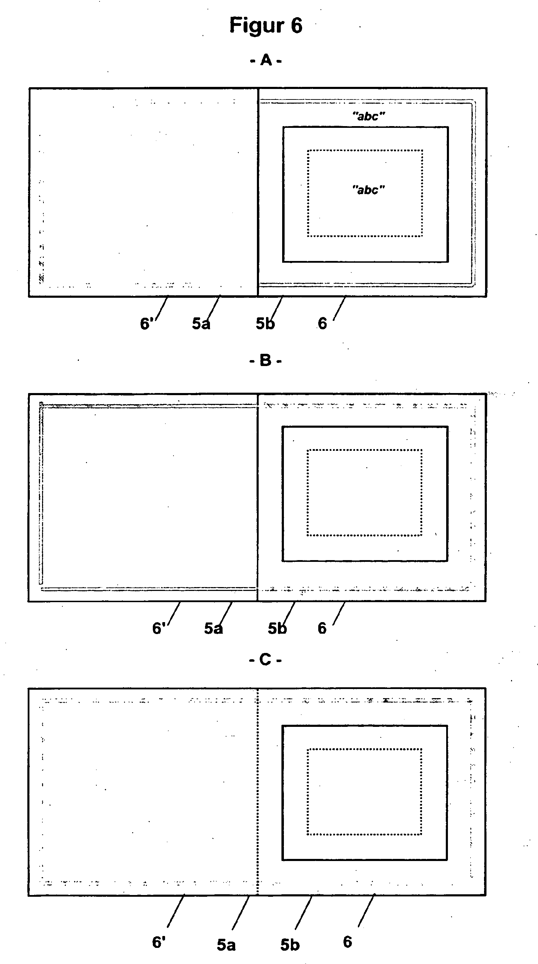

Figuren 6A bis C zeigen weitere bevorzugte Ausführungsformen der erfindungsgemäßen Verpackungen. Die Verpackung umfasst eine erste Außenwand (5a=6') und eine zweite Außenwand (5b=6). Im geschlossenen Zustand liegen die Außenwände (5a) und (5b) im wesentlichen deckungsgleich übereinander und bilden dadurch den Innenraum (1). In Figur 6A ist Außenwand (5a) aus einem transparenten Material gebildet, in Figur 6 B ist Außenwand (5b) aus einem transparenten Material gebildet und in Figur 6C sind sowohl Außenwand (5a) als auch Außenwand (5b) aus einem transparenten Material gebildet, was jeweils durch die graue Darstellung angedeutet wird. In Figur 6A sind ferner das Pflaster (2) und die Oberfläche (4) mit der Aufschrift "abc" bedruckt, wobei die Bedruckung "abc" im geschlossenen Zustand der Verpackung durch die transparente Außenwand (5a) hindurch von außen betrachtet werden kann.

Das Pflaster in der erfindungsgemäßen Verpackung ist wirkstoffhaltig. In der Therapie haben sich wirkstoffhaltige Pflaster bewährt, da sie eine schmerzlose, bequeme und einfache Verabreichung eines Wirkstoffes an den Patienten über einen längeren Zeitraum ermöglichen.

Üblicherweise umfasst ein wirkstoffhaltiges Pflaster wenigstens
a) eine für den Wirkstoff undurchlässige Trägerschicht.
b) eine klebefähige Schicht, welche die Trägerschicht zumindest teilweise bedeckt, und
c) ggf. eine Schutzfolle, welche die klebefähige Schicht zumindest teilweise bedeckt und davon abziehbar Ist; erfindungsgemäß übernimmt die Oberfläche der Außenwand der Verpackung diese Funktion (siehe oben).

Hinsichtlich des Aufbaus und der Funktionsweise kann bei wirkstoffhaltigen Pflastern zwischen membrangesieuerten und matrixgesteuerten Pflastern unterschieden werden.

Ein Reservoir-Pflaster umfasst üblicherweise einen auf der Haut selbstklebenden, flachen Beutel, welcher die Wirkstoffe dispergiert enthält. Der Hautseite zugewandt ist der Beutel mit einer für den Wirkstoff durchlässigen Membran ausgerüstet, welche die Wirkstofftreigabe steuert. Klebefähige Schicht und wirkstoffhattige Schicht (Reservoir) sind üblicherweise räumlich voneinander getrennte Untereinheiten eines Reservoir-Pflasters. Die klebefähige Schicht muss dann Ihrerseits zur Aufnahme des Wirkstoffs befähigt sein, denn der Wirkstoff muss die klebefähige Schicht durchdringen, um von der wirkstoffhaltigen Schicht zur Haut zu gelangen. Derartige Pflaster welsen somit gewissermaßen zwei wirkstoffhaltige Schichten auf, von denen eine Schicht zusätzlich klebefähig ist, die andere jedoch nicht.

Bei einem Matrix-Pflaster ist der Wirkstoff in einer Matrix eingebettet, wobei er in flüssigem, halbfestern oder festem Zustand dispergiert oder in gelöster Form vorliegen kann. Weist die Matrix gleichzeitig klebefähige Eigenschaften auf, so ist die klebefähige Schicht gleichzeitig auch die wirkstoffhaltige Schicht. Es ist aber auch möglich, dass die klebefähige Schicht und die wirkstoffhaltige Schicht - wie auch bei einem Reservoir-Pflaster - voneinander getrennt sind, wobei auch in diesem Fall die klebefähige Schicht zur Aufnahme des Wirkstoffs geeignet sein muss. In diesem Fall kann die klebefähige Schicht flächendeckend oder partiell, z.B. ringförmig auf der wirkstoffhaltigen Schicht bzw. der Membran des Reservoirs aufgebracht sein kann.

Durch die Zusammensetzung und Strukturierung der Matrix und/oder der Membran kann die Freisetzung des Wirkstoffs reguliert werden. Hinsichtlich weiterer Einzelheiten kann beispielsweise auf T.K. Gosh, Transdermal and Topical Drug Delivery Systems Es Into Practice, CRC Press, 1997; R.O. Potts et al., Mechanisms of Transdermal Drug Delivery (Drugs and the Pharmaceutical Sciences), Marcel Dekker, 1997; und R. Gumy et al, Dermal and Transdermal Drug Delivery. New insights and Perspectives, Wissenschaftliche VG., Stuttgart, 1998 verwiesen werden.

Damit ein Wirkstoff mit Hilfe eines wirkstoffhaltigen Pflasters transdermal verabreicht werden kann (bei dem wirkstoffhaltigen Pflaster handelt es sich dann um ein TTS), muss der Wirkstoff in ausreichender Menge durch die Epidermis der Haut, insbesondere auch das *Stratum Corneum* diffundieren, die einzelnen darunter liegenden Hautschichten passieren und vom Blutkreislauf aufgenommen werden. Ein wesentliches Problem stellt dabei häufig die nur geringe Durchlässigkeit der Haut für bestimmte Wirkstoffe dar. Je nach chemischer Struktur und Lipophile des Wirkstoffs kann es daher erforderlich sein, die transkutane Permeation des Wirkstoffs aus dem TTS durch Zusatz geeigneter Hilfsstoffe zu verbessern.

Neben den gewünschten pharmakokinetischen Parametern muss ein wirkstoffhaltiges Pflaster eine ausreichende Hautverträglichkeit aufweisen. Die Haftung des wirkstoffhaltigen Pflasters auf der Haut wird üblicherweise mit Hilfe druckempfindlicher Klebstoffe (medizinischer Haftkleber) erreicht. Um die chemischen und/oder physikalischen Eigenschaften, z.B. die Kohärenz oder die Klebrigkeit medizinischer Haftkleber zu modifizieren und in Abhängigkeit von den jeweiligen Erfordernissen zu optimieren, werden üblicherweise bestimmte Hilfs- oder Zusatzstoffe zugesetzt, insbesondere Weichmacher oder Klebrigmacher (*tackifier*)*.* Diese Hilfs- oder Zusatzstoffe sollten jedoch mit den übrigen verwendeten Materialien kompatibel sein. So sollten die genannten Hilfs- oder Zusatzstoffe die chemische Stabilität der enthaltenen Wirkstoffe oder deren Freisetzung nicht negativ beeinflussen. Häufig kann es auch vorteilhaft sein, wenn die Polymermatrix eines wirkstoffhaltigen Haftklebers überwiegend lipophile Eigenschaften aufweist, damit sie eine hohe Beladungskapazität für lipophilie Wirkstoffe besitzt.

Bei dem wirkstoffhaltigen Pflaster in der erfindungsgemäßen Verpackung handelt es sich vorzugsweise um ein Pflaster herkömmlichen Aufbaus, d.h. das Pflaster umfasst neben einer Trägerschicht zumindest eine klebefähige Schicht. Bevorzugte Ausführungsformen derartiger wirkstoffhaltiger Pflaster werden nachfolgend beschrieben:

Die klebefähige Schicht der wirkstoffhaltigen Pflaster basiert bevorzugt auf einem druckempfindlichen Klebstoff. Der druckempfindliche Klebstoff enthält bevorzugt wenigstens ein Polymer, vorzugsweise ausgewählt aus der Gruppe bestehend aus Polyacrylaten, Polyvinylethern, Polyvinylalkoholen, Polyisobutylenen, Acrylat-Copolymerisaten, Ethylen-Vinylacetat-Copolymerisaten, Polyurethanen, Styrol-Isopren-Copolymerisaten, Styrol-Butadien-Copolymerisaten, Cellulose-Derivaten, Silikonen, Kautschuken, Harzen und ggf. hydrierten Estern des Kolophoniums.

Geeignete Cellulose-Derivate sind z.B. Ethylcellulose, Propylcellulose, Hydroxyethylcellulose.Hydroxypropylcellulose und Hydroxypropylmethylcellulose.

Geeignete Polyacrylate sind z.B. (Co-)Polymerisate von Acrylaten, Alkylacrylaten, Methacrylaten, und/oder Alkylmethacrylaten, die ggf. mit weiteren ungesättigten Monomeren wie Acrylamid, Dimethylacrylamid, Dimethylaminoethylacrylat, Hydroxyethylacrylat, Hydroxypropylacrylat, Methoxyethylacrylat, Methoxyethylmethacrylat, Acrylnitril und/oder Vinylacetat copolymerisiert sind.

Im Sinne der Beschreibung bedeutet "(meth)acryl" "acryl" und/oder "methacryl".

Besonders geeignete (Meth)acrylsäureester sind solche, die lineare, verzweigte oder cyclische eliphatische C₁₋C₂₋Substituenten ohne sonstige funktionelle Gruppen tragen. Zu dieser Gruppe zählen insbesondere Methyl(meth)acrylat, Ethyl(meth)-acrylat, Isopropyl(meth)acrylat, n-Butyl(meth)acrylat, Isobutyl(meth)acrylat, sec.-Butyl(meth)acrylat, tert.-Butyl(meth)acrylat, 2-Ethylhexyl(meth)acrylat, Cyclohexyl-(meth)acrylat und Isobornyl(meth)acrylat.

Doch können auch (Meth)acrylsäureester in dem zur Herstellung des Polyacrylats verwendeten Monomerengemisch enthalten sein, die funktionelle Gruppen tragen. Hierunter sind u.a. hydroxylgruppenhaltige Ester zu verstehen, wie 2-Hydroxyethyl-(meth)acrylat und 3-Hydroxypropyl(meth)acrylat. Aber auch Stoffe wie Acrylnitril, Vinylacetat, Methoxyethyl(meth)acrylat, Acrylamid, Dimethylacrylamid, Dimethylaminoethylacrylat etc. können als funktionelle Gruppen enthaltende (Meth)acrylsäureester verstanden werden. Der Anteil von (Meth)acrylsäureestern, die derartige funktionelle Gruppen enthalten, soll dabei im Monomerengemisch kleiner oder gleich 10 Gew.-%, vorzugsweise weniger als 2 Gew.-%, noch bevorzugter weniger als 0,2 Gew.-%, betragen. Besonders bevorzugt ist ein Monomerengemisch, das keine funktionelle Gruppen enthaltende (Meth)acrylsäureester enthält.

Es kann jedoch auch Vinylacetat als Co-Monomer zusammen mit mindestens einem Monomeren aus der Gruppe der (Meth)acrylsäureester zur Herstellung des Polyacrylats verwendet werden. Der Anteil des Vinylacetats in dem zur Herstellung dieses Polyacrylats verwendeten Monomerengemisch sollte unterhalb von 50 Gew.-%, vorzugsweise unterhalb von 25 Gew. -% liegen. Besonders bevorzugt ist ein Vinylacetatgehalt zwischen 0 und 5,0 Gew.-%. Besonders bevorzugt sind Acrylat-Vinylacetat-Copolymerisate, welche beispielsweise unter der Bezeichnung DURO-TAK^{®} im Handel erhältlich sind. Besonders bevorzugt werden diese Copolymerisate in Kombination mit Polyacrylaten, beispielsweise Carbopol^{®}, insbesondere Carbopol^{®} 980, oder mit Hydroxypropylcellulose eingesetzt.

Beispiele für geeignete Harze sind Ester von Glycinen, Glycerin oder Pentaerythrit, Kohlenwassertoffharze und Polyterpene.

Weiterhin eignen sich Silikon-Klebstoffe, wie z.B. ggf. vernetzte Polydimethylsiloxane.

Der druckempfindliche Klebstoff enthält vorzugsweise Klebrigmacher, welche sich strukturell vorzugsweise vom tricyclo-[5.2.1.0^{2,6}]Decan ableiten. Besonders bevorzugt sind *bis*-(Hydroxymethyl)-tricyclo-[5.2.1.0^{2,6}]decan, *bis*-(Aminomethyl)-tricyclo-[5.2.1.0^{2.6}]decan, und *bis*-Formyl-tricyclo-[5.2.1.0^{2.6}]decan, insbesondere die Derivate des tricyclo-[5.2.1.0^{2,6}]Decans, welche die beiden Substituenten in den Positionen 3(4) und 8(9) tragen. Andere geeignete Klebrigmacher sind Derivate von Kohlenhydraten, insbesondere SAIB.

Der druckempfindliche Klebstoff kann weitere Hilfsstoffe enthalten, welche ihn zur Anwendung auf der menschlichen Haut besonders geeignet machen können. Beispiele für solche Hilfsstoffe sind hautpermeationsfördemde Substanzen, Lösungsmittel, Lösungsvermittler, Emulgatoren, Vernetzer, Stabilisatoren, Füllstoffe (z.B. Zinkoxid, Silica) Konservierungsmittel, Farbstoffe, hautglättende Mittel, Duftstoffe, zur Reduzierung oder Verhinderung von Hautirritationen auf die Hautoberfläche übertragbare Verbindungen, Verdickungsmittel, Weichmacher, Klebrigmacher, etc. Solche Hilfsstoffe sind dem Fachmann bekannt. In diesem Zusammenhang kann beispielsweise verwiesen werden auf H.P. Fiedler, Lexikon der Hilfsstoffe für Pharmazie, Kosmetik und angrenzende Gebiete, Editio Cantor Aulendorff, 2002; R. Niedner et al., Dermatika: therapeutischer Einsatz, Pharmakologie und Pharmazie, Wiss. Verl.-Ges. 1992. Konkrete Beispiele für die vorstehend genannten Substanzklassen werden nachfolgend auch im Zusammenhang mit weiteren Aspekten der Erfindung beschrieben.

Der Wirkstoff kann in der klebefähigen Schicht (*drug in adhesive*) und/oder einer weiteren Schicht, beispielsweise in einer von der klebefähigen Schicht separierten Matrix (*drug in matrix*)*,* enthalten sein.

In einer bevorzugten Ausführungsform ist der Wirkstoff wenigstens teilweise in der klebefähigen Schicht enthalten. In einer bevorzugten Ausführungsform ist der Wirkstoff wenigstens teilweise in einer Matrix eingebettet, wobei die Matrix ihrerseits Bestandteil der klebefähigen Schicht sein oder eine eigenständige Schicht bilden kann.

In einer bevorzugten Ausführungsform handelt es sich um ein Matrix-Pflaster. Das Matrix-Pflaster umfasst bevorzugt eine Trägerschicht, von der eine Oberfläche an die Matrix angrenzt, welche den Wirkstoff enthält (wirkstoffhaltige Schicht), und eine klebefähige Schicht. Die klebefähige Schicht bedeckt vorzugsweise die gesamte Fläche der Trägerschicht. Die dazwischen liegende Matrix bedeckt vorzugsweise nur einen Teil der Fläche der Trägerschicht, so dass ein äußerer Rand der Trägerschicht verbleibt, welcher zwar mit der klebefähigen Schicht, nicht jedoch mit der Matrix bedeckt ist. Um die Migration des Wirkstoffs in die Trägerschicht zu minimieren, kann es vorteilhaft sein, zwischen Trägerschicht und Matrix eine Sperrschicht aus einem geeigneten Material einzufügen.

Bei dem Matrix-Pflaster ist der Wirkstoff zumindest zum Teil in einer Matrix eingebettet (wirkstoffhaltige Schicht). Die Matrix basiert vorzugsweise auf lipophilen oder hydrophilen, vorzugsweise vernetzbaren Polymeren. Hydrophile matrixbildende Schichten können wasserhaltig sein, wobei es sich in einem solchen Fall vorzugsweise um Gele handelt. Vorzugsweise basiert die Matrix auf wenigstens einem Polymer ausgewählt aus der Gruppe umfassend Cellulose-Derivate, wie z.B. Cellulose-Ether, besonders bevorzugt Methylcellulose, Ethylcellulose, Propylcellulose, Hydroxyethylcellulose, Hydroxypropylcellulose, Hydroxypropylmethylcellulose, und/oder Carboxymethylcellulose; Polyethylene; chlorierte Polyethylene, wie Polyvinylchloride oder Polyvinylidenchloride; Polypropylene; Polyurethane; Polycarbonate; Polyacrylsäureester; Polyacrylate; Polymethacrylate; Polyvinylalkohole; Polyvinylpyrrolidone; Polyethylenterephthalate; Polytetrafluorethylene; Ethylen-Propylen-Copolymere; Ethylen-Ethylacrylat-Copolymere; Ethylen-Vinylacetat-Copolymere; Ethylen-Vinylalkohol-Copolymere; Ethylen-Vinyloxyethanol-Copolymere; Vinylchlorid-Vinylacetat-Copolymere; Vinylpyrrolidon-Ethylen-Vinylacetat-Copolymere; Kautschuke; synthetische Homo-, Co- oder Blockpolymere aus Butadien und/oder Styrol, wie Styrol-Isopren-Styrol-Blockcopolymere; Silikone; Silkon-Derivate, besonders bevorzugt Siloxan-Methacrylat-Copolymere, Polyvinylether, Polyester, und/oder Polysaccharide, bzw. deren Mischungen. Besonders bevorzugt sind Polyacrylate oder Acrylat Copolymerisate, insbesondere solche, wie sie vorstehend im Zusammenhang mit den Polymeren des druckempfindlichen Klebstoffs beschrieben sind.

In einer weiteren bevorzugten Ausführungsform handelt es sich bei dem wirkstoffhaltigen Pflaster um ein sog. "monolithisches *drug-in-adhesive"* Pflaster. In diesem Fall ist der Wirkstoff in der klebefähigen Schicht enthalten (*drug in adhesive*)*.* Ein derartiges Pflaster umfasst bevorzugt eine Trägerschicht und eine klebefähige Schicht, welche den Wirkstoff enthält. Die wirkstoffhaltige Schicht kann zusätzlich matrixbildende Polymere enthalten, so dass der Wirkstoff in einer Matrix eingebettet ist (*drug in matrix*)*,* welche ihrerseits Bestandteil der klebefähigen Schicht ist (*drug in adhesive*). Zur Herstellung einer solchen klebefähigen Schicht können die Bestandteile des druckempfindlichen Klebstoffs als klebefähige Komponente mit den vorstehend aufgeführten Matrixmaterialien in bekannten Mengen gemischt werden und zur Herstellung des wirkstoffhaltigen Matrixbereichs eine physiologisch wirksame Substanz hinzugefügt werden. Nach dem Auftrag kann das Matrixmaterial, wenn nötig, auch noch vernetzt werden. Der in der Matrix vorliegende Wirkstoff oder die in der Matrix vorliegenden Wirkstoffe können flüssig,halbfest oder fest im dispergierten Zustand vorliegen oder als entsprechende Formulierung unter Hinzufügung von üblichen Hilfsstoffen als Wirkstoffformulierung eingearbeitet sein.

Sofern das wirkstoffhaltige Pflaster keine vollflächige klebefähige Schicht aufweist, kann es auch so gestaltet sein, dass der druckempfindliche Klebstoff nur in den Randzonen der wirkstoffhaltigen Schicht vorliegt, wobei diese Randzonen vorzugsweise keinen Wirkstoff enthalten.

In einer weiteren bevorzugten Ausführungsform ist das wirkstoffhaltige Pflaster mehrschichtig, wobei der Wirkstoff zumindest zum Teil in der klebefähigen Schicht enthalten ist. Bei dieser Ausführungsform ist wenigstens eine selektiv-permeable Membran zwischen zwei klebefähigen Schichten vorhanden, wobei beide klebefähigen Schichten wirkstoffhaltig sind (vgl. T.A. Petersen et al., Intern. Symp. Control. Rel. Bioact. Mater., 21:477-478). Geeignete selektiv-permeable Membranen sind kommerziell erhältlich (vgl. z.B. R.E. Kesting, Synthetic Polymer Membranes, McGraw Hill; J.D. Ferry, Ultrafiltration Membranes, Chemical Review, 18, 373).

In einer anderen bevorzugten Ausführungsform handelt es sich bei dem wirkstoffhaltigen Pflaster um ein Reservoir-Pflaster. Das Reservoir-Pflaster umfasst vorzugsweise eine Trägerschicht und ein Reservoir, welches den Wirkstoff in gelöster oder suspendierter Form enthält, wobei das Reservoir zumindest auf der Seite, welche bei der Applikation der Haut zugewandt ist, mit einer selektiv-permeablen Membran umgeben ist. Die Trägerschicht weist vorzugsweise eine gewisse mechanische Beständigkeit und ggf. Seitenwände auf. Die Kapazität des Reservoirs ist von der räumlichen Ausdehnung des so gebildeten Hohlraums abhängig. Die klebefähige Schicht kann sich über die gesamte Fläche des Reservoir-Pflasters, welche bei der Applikation der Haut zugewandt ist, erstrecken. Es ist aber auch möglich, dass nur Teile dieser Fläche mit der klebefähigen Schicht bedeckt sind, etwa nur die der Haut zugewandte Fläche der Trägerschicht, welche nicht mit dem Reservoir bzw. der selektiv-permeablen Membran bedeckt ist.

Handelt es sich um ein Reservoir-Pflaster, so liegt der Wirkstoff in einem Reservoir vor. Das Reservoir wird vorzugsweise von einer selektiv-permeablen Membran umschlossen oder zumindest auf einer Seite von dieser begrenzt. Als Membranmaterialien sind die vorstehend aufgeführten Polymere geeignet, die auch als Matrixmaterial zum Einsatz kommen können. Vorzugsweise basiert die Membran auf wenigstens einem Polymer ausgewählt aus der Gruppe bestehend aus Polyethylenen, Polypropylenen, Polyvinylacetaten, Polyamiden, Ethylen-Vinylacetat-Copolymeren, Polyethylenterephthalaten und Silikonen. Mit Hilfe der Reservoir-Membran kann aus dem Reservoir eine kontrollierte Freisetzung des Wirkstoffes erzielt werden.

Das Reservoir kann vorzugsweise zwischen einer Deckschicht und der klebefähigen Schicht angeordnet sein. Das Reservoir kann ggf. auch als ein Vlies, Gewebe oder dergleichen ausgebildet sein, welches mit der Wirkstoff-Formulierung getränkt ist.

Das Reservoir enthält wenigstens einen Wirkstoff, vorzugsweise als Lösung, die durch die das Reservoir umschließende Membran ungehindert diffundieren kann. Die Lösung kann eine erhöhte Viskosität aufweisen, beispielsweise kann es sich um eine Hydrogel oder um ein Lipogel handeln. Ein Hydrogel enthält vornehmlich hydrophile Substanzen, jedoch nicht notwendigerweise Wasser. Als Lösungsmittel sind solche Lösungsmittel geeignet, in denen sich der Wirkstoff ausreichend löst, so dass dadurch eine Ausfällung der Wirkstoffe vermieden wird.

Sind die wirkstoffhaltigen Pflastern zur transdermalen Verabreichung des enthaltenen Wirsktoffs bestimmt, so können als übliche Hilfsstoffe Verbindungen eingesetzt werden, die den transdermalen Transport der Wirkstoffe verstärken bzw. erleichtern (hautpermeationsfördernde Substanzen). Es können die dem Fachmann für den jeweiligen, transdermal zu verabreichenden Wirkstoff bekannten hautpermeationsfördernden Substanzen verwendet werden. In diesem Zusammenhang kann beispielsweise verwiesen werden auf D.S. Hsieh, Drug Permeation Enhancement: Theory and Applications (Drugs and the Pharmaceutical Sciences: a Series of Textbooks and Monographs), Marcel Dekker, 1994 und E.W. Smith et al., Percutaneous Penetration Enhancers, CRC Press, 1995.

Sämtliche Materialien, die zum Aufbau der transdermalen Systeme verwendet werden, insbesondere aber diejenigen Materialien, die mit der Haut in Berührung kommen,sollten hautverträglich und physiologisch unbedenklich sein.

Als Stabilisatoren, Emulgatoren und Verdickungsmittel werden die dem Fachmann bekannten Hilfsstoffe verwendet. Diese Hilfsstoffe können mit den Wirkstoffen gemischt oder in einer separaten Schicht zur wirkstoffhaltigen Schicht vorliegen.

Die Schichtdicke der klebefähigen Schicht des wirkstoffhaltigen Pflasters beträgt vorzugsweise 3 bis 100 µm, bevorzugt 5 bis 90 µm und insbesondere 10 bis 80 µm.

Das wirkstoffhaltige Pflaster hat vorzugsweise eine Gesamtfläche (Kontaktfläche zur Haut bei bestimmungsgemäßer Anwendung) von 0,5 bis 200 cm², bevorzugter 20 bis 150 cm².

Bei dem wirkstoffhaltigen Pflaster grenzt die klebefähige Schicht vorzugsweise unmittelbar an eine Trägerschicht an.

Die Trägerschicht umfasst vorzugsweise ein flexibles, dehnbares, atmungsaktives, strapazierfähiges Material und kann gefärbt, beispielsweise hautfarben sein. Die Trägerschicht weist vorzugsweise eine solche Dicke auf, dass das wirkstoffhaltige Pflaster eine ausreichende mechanische Stabilität aufweist. Vorzugsweise ist die Trägerschicht wirkstoffundurchlässig, d.h. zumindest in dem Bereich, in dem die Trägerschicht an eine wirkstoffhaltige Matrix bzw. ein Wirkstoffreservoir angrenzt, ist sie für den Wirkstoff undurchlässig. Ist die Trägerschicht als solche nicht wirkstoffundurchlässig, sollte zwischen der wirkstoffhaltigen Schicht, d.h. der Matrix bzw. dem Wirkstoffreservoir, und der Trägerschicht eine wirkstoffundurchlässige Sperrschicht angeordnet sein. Diese Sperrschicht basiert dann vorzugsweise gleich oder verschieden auf einem oder mehreren Materialien, wie nachfolgend für die Trägerschicht ausgeführt:

Als Beispiele für geeignete Materialien der Trägerschicht können insbesondere genannt werden Polyester bzw. Copolyester, bevorzugt Polyethylenterephthalate; Polyolefine bzw. Olefin-Copolymerisate, bevorzugt Polyethylene, Polypropylene oder Polybutylene; Polycarbonate; Polyethylenoxide; Polyurethane; Polystyrole; Polyamide bzw. Copolyamide; Polyimide; Polyvinylacetate; Polyvinylchloride; Polyvinylidenchloride;Copolymerisate,bevorzugt Acrylnitril-Butadien-Styrol-Terpolymere oder Ethylen-Vinylacetat-Copolymerisate, Papier, Textilien und deren Mischungen. Besonders bevorzugt basiert die Trägerschicht auf einem Polyester, insbesondere auf einem Polyethylenterephthalat, beispielsweise auf Hostaphan^{®} RN. Die Trägerschicht kann auch metallisiert und/oder pigmentiert oder als eine Schichtfolge aus den vorstehend genannten Materialien und einer Metallfolie, besonders bevorzugt einer Folie aus Aluminium, bestehen.

In einer bevorzugten Ausführungsform ist die Trägerschicht mit einem anorganischorganischen Hybridpolymer beschichtet. Anorganisch-organische Hybridpolymere, sogenannte Ormocere, sind im Stand der Technik bekannt (vgl. z.B. EP-A 0 358 011; EP-A 0 373 451; EP-A 0 610 831; EP-B 0 644 908; EP-A 0 792846; EP-A 0 934 989).

In einer bevorzugten Ausführungsform weist die Trägerschicht eine Schichtdicke im Bereich von 5,0 bis 125 µm, bevorzugter 10 bis 115 µm noch bevorzugter 25 bis 100 µm, am bevorzugtesten 35 bis 95 µm und insbesondere 50 bis 85 µm auf.

Bevorzugt bildet die Trägerschicht eine der beiden Oberflächenschichten des wirkstoffhaltigen Pflasters, d.h. ausgehend von der vorzugsweise unmittelbar an die Trägerschicht angrenzenden klebefähige Schicht enthält das wirkstoffhaltige Pflaster jenseits der Trägerschicht bevorzugt keine weitere Schicht.

In einer weiteren bevorzugten Ausführungsform umfasst das wirkstoffhaltige Pflaster mehrere vereinzelbare Untereinheiten, welche ihrerseits in Form einer eigenständigen Einheit als wirkstoffhaltiges Pflaster verwendet werden können, wobei die einzelnen Untereinheiten miteinander über vorzugsweise wirkstofffreie Randbereiche, in denen Trennungsmöglichkeiten vorgesehen sind, zu einer zusammenhängenden Einheit verbunden sind. Vorzugsweise liegen linienförmige Perforationen zur Trennung der vereinzelbaren Untereinheiten von der zusammenhängenden Einheit vor. Weiter bevorzugt sind Schneidemarkierungen auf der Trägerschicht, welche die Trennmöglichkeit der vereinzelbaren Untereinheiten von der Einheit vorgeben. Die Trennmöglichkeiten sind jeweils vorzugsweise so angeordnet, dass sie eine Abtrennung jeder zusammenhängenden Untereinheit ermöglichen. Die einzelnen Untereinheiten können gleich oder verschieden sein. Sind die Untereinheiten verschieden, so können sie beispielsweise den darin enthaltenen Wirkstoff in verschiedenen Dosierungen enthalten bzw. mit verschiedenen Freisetzungsraten abgeben.

Die Herstellung des wirkstoffhaltigen Pflasters kann nach bekannten Herstellungsverfahren erfolgen und übliche Verfahrensschritte umfassen, wie Laminieren, Coextrudieren, Stanzen, Delaminieren, Abwickeln, Schneiden, Wiederaufwickeln, Montieren oder Dosieren (Verpackungs-Rundschau 4/2002, 83-84).

## Patentansprüche

1. Verpackung, in deren Innenraum (1) mindestens ein wirkstoffhaltiges Pflaster (2) angeordnet ist, welches über seine klebefähige Schicht (3) an einer dem Innenraum (1) zugewandten Oberfläche (4) einer Außenwand (5) der Verpackung haftet und davon ablösbar ist, **dadurch gekennzeichnet, daß** die Verpackung wenigstens in einem Bereich transparent ist und auf das Pflaster (2) Informationen als Kennzeichnungen aufgedruckt sind, welche durch die transparente Ausgestaltung der Verpackung nach außen sichtbar sind.

2. Verpackung nach Anspruch 1, **dadurch gekennzeichnet, dass** die gesamte Fläche des Pflasters (2), welche seiner klebefähigen Schicht (3) abgewandt ist, durch den transparenten Bereich der Verpackung hindurch mit bloßem Auge betrachtet werden kann.

3. Verpackung nach Anspruch 1 oder 2, **dadurch gekennzeichnet, dass** der transparente Bereich auf seiner dem Innenraum (1) der Verpackung zugewandten Oberfläche mit einer klebefähigen Schicht ausgerüstet ist, welche an derjenigen Fläche des Pflasters (2) haftet, die dessen klebefähiger Schicht (3) abgewandt ist.

4. Verpackung nach Anspruch 3, **dadurch gekennzeichnet, dass** die klebefähige Schicht (3) des Pflasters (2) wirkstoffhaltig ist.

5. Verpackung nach einem der vorstehenden Ansprüche, **dadurch gekennzeichnet, dass** die Außenwand (5) ein- oder mehrteilig ausgebildet ist.

6. Verpackung nach einem der vorstehenden Ansprüche, **dadurch gekennzeichnet, dass** die Außenwand (5) aus einer ein- oder mehrschichtigen Folie gebildet wird.

7. Verpackung nach einem der vorstehenden Ansprüche, **dadurch gekennzeichnet, dass** die Außenwand (5) zwei im wesentlichen spiegelsymmetrische Teilbereiche (6) und (6') umfasst.

8. Verpackung nach Anspruch 7, **dadurch gekennzeichnet, dass** Teilbereich (6) und/oder Teilbereich (6') wenigstens in einem Teilbereich aus einem transparenten Material gebildet ist.

9. Verpackung nach Anspruch 7 oder 8, **dadurch gekennzeichnet, dass** die Außenwand (5) einteilig ausgebildet ist und die Teilbereiche (6) und (6') über eine Falz (7) aneinander angrenzen.

10. Verpackung nach einem der Ansprüche 7 bis 9, **dadurch gekennzeichnet, dass** die Teilbereiche (6) und (6') im wesentlichen deckungsgleich übereinander liegen und **dadurch** den Innenraum (1) bilden.

11. Verpackung nach Anspruch 10, **dadurch gekennzeichnet, dass** der Randbereich der dem Innenraum (1) zugewandten Oberfläche des Teilbereichs (6) mit dem Randbereich der dem Innenraum (1) zugewandten Oberfläche des Teilbereichs (6') über eine Siegelnaht (8) verbunden ist.

12. Verpackung nach einem der vorstehenden Ansprüche, **dadurch gekennzeichnet, dass** das Pflaster (2) wirkstoffhaltig und die klebefähige Schicht (3) unterteilt ist in einen wirkstofffreisetzenden Bereich (3a) und einen wirkstofffreien Bereich (3b).

13. Verpackung nach Anspruch 12, **dadurch gekennzeichnet, dass** der wirkstofffreie Bereich (3b) den wirkstoffhaltigen Bereich (3a) am äußeren Rand der klebefähigen Schicht (3) umgibt.

14. Verpackung nach einem der vorstehenden Ansprüche, **dadurch gekennzeichnet, dass** die Außenwand (5) wenigstens eine Lasche (9) zum Öffnen der Verpackung aufweist.

## Claims

1. A package, in the interior (1) of which is arranged at least one active ingredient-containing patch (2), which adheres by means of its adhesive layer (3) to a surface (4), which faces the interior (1), of an outer wall (5) of the package and is detachable therefrom, **characterised in that** the package is transparent at least in one zone and information is printed as markings on the patch (2) which are visible from the outside thanks to the transparent nature of the package.

2. A package according to claim 1, **characterised in that** the entire face of the patch (2) remote from its adhesive layer (3) can be observed by the naked eye through the transparent zone of the package.

3. A package according to claim 1 or claim 2, **characterised in that** the transparent zone is provided on its surface facing the interior (1) of the package with an adhesive layer which adheres to that face of the patch (2) which is remote from the adhesive layer (3) thereof.

4. A package according to claim 3, **characterised in that** the adhesive layer (3) of the patch (2) contains active ingredient.

5. A package according to any one of the preceding claims, **characterised in that** the outer wall (5) is of single part or multipart construction.1

6. A package according to any one of the preceding claims, **characterised in that** the outer wall (5) is formed from a single layer or multilayer film.

7. A package according to any one of the preceding claims, **characterised in that** the outer wall (5) comprises two substantially mirror-symmetrical sub-zones (6) and (6').

8. A package according to claim 7, **characterised in that** sub-zone (6) and/or sub-zone (6') is formed at least in a sub-zone from a transparent material.

9. A package according to claim 7 or claim 8, **characterised in that** the outer wall (5) is of single part construction and the sub-zones (6) and (6') adjoin one another via a fold (7).

10. A package according to any one of claims 7 to 9, **characterised in that** the sub-zones (6) and (6') lie substantially congruently one on the other and in this manner form the interior (1).

11. A package according to claim 10, **characterised in that** the peripheral zone of the surface of sub-zone (6) facing the interior (1) is joined via a seal seam (8) to the peripheral zone of the surface of sub-zone (6') facing the interior (1).

12. A package according to any one of the preceding claims, **characterised in that** the patch (2) contains active ingredient and the adhesive layer (3) is subdivided into an active ingredient-releasing zone (3a) and an active ingredient-free zone (3b).

13. A package according to claim 12, **characterised in that** the active ingredient-free zone (3b) surrounds the active ingredient-containing zone (3a) at the outer edge of the adhesive layer (3).

14. A package according to any one of the preceding claims, **characterised in that** the outer wall (5) comprises at least one tab (9) for opening the package.

## Revendications

1. Emballage, dans l'espace interne (1) duquel est disposé au moins un pansement (2) contenant une substance active, qui par sa couche adhésive (3) adhère à une surface (4) d'une paroi extérieure (5) de l'emballage tournée vers l'espace interne (1), et qui peut en être décollé, **caractérisé en ce que** l'emballage est au moins transparent dans une région et des informations sous forme d'inscriptions sont imprimées sur le pansement (2), lesquelles sont visibles de l'extérieur du fait de la configuration transparente de l'emballage.

2. Emballage selon la revendication 1, **caractérisé en ce que** toute la surface du pansement (2) qui est tournée à l'opposé de sa couche adhésive (3), peut être vue à l'oeil nu à travers la région transparente de l'emballage.

3. Emballage selon la revendication 1 ou 2, **caractérisé en ce que** la région transparente est équipée sur sa surface tournée vers l'espace intérieur (1) de l'emballage d'une couche adhésive qui adhère à la face du pansement (2) qui est opposée à sa couche adhésive (3).

4. Emballage selon la revendication 3, **caractérisé en ce que** la couche adhésive (3) du pansement (2) contient une substance active.

5. Emballage selon l'une quelconque des revendications précédentes, **caractérisé en ce que** la paroi extérieure (5) est réalisée d'une seule pièce ou en plusieurs parties.

6. Emballage selon l'une quelconque des revendications précédentes, **caractérisé en ce que** la paroi extérieure (5) est formée d'un film monocouche ou multicouche.

7. Emballage selon l'une quelconque des revendications précédentes, **caractérisé en ce que** la paroi extérieure (5) comprend deux régions partielles (6) et (6') essentiellement à symétrie spéculaire.

8. Emballage selon la revendication 7, **caractérisé en ce que** la région partielle (6) et/ou la région partielle (6') sont formées au moins dans une région partielle d'un matériau transparent.

9. Emballage selon la revendication 7 ou 8, **caractérisé en ce que** la paroi extérieure (5) est réalisée d'une seule pièce et les régions partielles (6) et (6') sont adjacentes l'une à l'autre au niveau d'un pli (7).

10. Emballage selon l'une quelconque des revendications 7 à 9, **caractérisé en ce que** les régions partielles (6) et (6') sont disposées essentiellement en alignement l'une au-dessus de l'autre et forment ainsi l'espace interne (1).

11. Emballage selon la revendication 10, **caractérisé en ce que** la région de bord de la surface de la région partielle (6) tournée vers l'espace interne (1) est connectée à la région de bord de la surface de la région partielle (6') tournée vers l'espace interne (1) par le biais d'un joint de scellage (8).

12. Emballage selon l'une quelconque des revendications précédentes, **caractérisé en ce que** le pansement (2) contient une substance active et la couche adhésive (3) est divisée en une région libérant la substance active (3a) et une région exempte de substance active (3b).

13. Emballage selon la revendication 12, **caractérisé en ce que** la région exempte de substance active (3b) entoure la région contenant la substance active (3a) au niveau du bord extérieur de la couche adhésive (3).

14. Emballage selon l'une quelconque des revendications précédentes, **caractérisé en ce que** la paroi extérieure (5) présente au moins une languette (9) pour l'ouverture de l'emballage.
